# EUROPÄISCHE PATENTSCHRIFT

(11) **EP 1 368 118 B1**
(45) Veröffentlichungstag und Bekanntmachung des Hinweises auf die Patenterteilung: **17.05.2006**
(21) Anmeldenummer: 02732454.0
(22) Anmeldetag: 01.03.2002
(51) Int. Cl.: B01J 29/89

(54) **FORMKÖRPER UND VERFAHREN ZU DESSEN HERSTELLUNG**
SHAPED BODY AND METHOD FOR PRODUCING THE SAME
CORPS MOULE ET PROCEDE PERMETTANT DE LE PRODUIRE

(30) Priorität: 02.03.2001 DE 10110139
(43) Veröffentlichungstag der Anmeldung: 10.12.2003
(73) Patentinhaber: BASF Aktiengesellschaft, 67056 Ludwigshafen (DE)
(72) Erfinder: MÜLLER, Ulrich, 67435 Neustadt (DE); SENK, Rainer, 67069 Ludwigshafen (DE); HARDER, Wolfgang, 69469 Weinheim (DE); RUDOLF, Peter, 68526 Ladenburg (DE); RIEBER, Norbert, 68259 Mannheim (DE)
(74) Vertreter: Isenbruck, Günter
(86) Internationale Anmeldenummer: PCT/EP2002/002278
(87) Internationale Veröffentlichungsnummer: WO 2002/085513

(56) Entgegenhaltungen:
- EP-A- 1 138 387
- WO-A-94/29408
- WO-A-97/47614
- WO-A-98/55229

## Beschreibung

Die vorliegende Erfindung betrifft einen mindestens einen Zeolithen enthaltenden Formkörper, ein Verfahren zu dessen Herstellung, sowie dessen Verwendung als Katalysator in Umsetzungen von organischen Verbindungen, insbesondere zur Epoxidation von organischen Verbindungen mit mindestens einer C-C-Doppelbindung. Der hierin beschriebene Formkörper weist hohe Abriebfestigkeit, ausgezeichnete mechanische Eigenschaften, sowie eine gegenüber den bekannten Katalysatoren auf Zeolith-Basis nochmals verbesserte Performance, insbesondere bei der Epoxidation von organischen Verbindungen auf.

Abriebfeste Formkörper aus katalytisch aktiven Massen werden in vielen chemischen Verfahren eingesetzt, insbesondere bei Verfahren unter Verwendung eines Festbetts. Demgemäß existiert eine immense Fülle von Literatur zu diesem Thema. Über den Einsatz von Katalysatoren auf der Basis poröser oxidischer Materialien, wie z.B. Zeolithe und speziell bezüglich der Verformung derartiger Materialien existiert bedeutend weniger Literatur.

In der Regel wird zur Herstellung von Festkörpern die katalytisch aktive Masse, d.h. das poröse oxidische Material mit einem Bindemittel, einer organischen viskositätssteigernden Verbindung und einer Flüssigkeit zum Anteigen der Masse versetzt und in einer Misch- oder Knetvorrichtung oder einem Extruder verdichtet. Anschließend wird die daraus resultierende plastische Masse verformt, insbesondere unter Verwendung einer Strangpresse oder eines Extruders und die resultierenden Formkörper getrocknet und calciniert.

Einen Überblick über den einschlägigen Stand der Technik gibt die WO 98/55229 und der darin zitierte Stand der Technik.

Als Bindemittel werden dabei eine Reihe von anorganischen Verbindungen benutzt. Auch diesbezüglich wird auf obigen Stand der Technik verwiesen.

Die WO 98/55229 selbst betrifft einen mindestens ein poröses oxidisches Material enthaltenden Formkörper, der erhältlich ist durch ein Verfahren, das die folgenden Stufen umfaßt:
(I) Versetzen eines Gemischs enthaltend ein poröses oxidisches Material oder ein Gemisch aus zwei oder mehr davon mit einer Mischung enthaltend mindestens einen Alkohol und Wasser, und
(II) Kneten, Verformen, Trocknen und Calcinieren des gemäß Stufe (I) versetzten Gemischs,
sowie ein
Verfahren zur Herstellung eines mindestens ein poröses oxidisches Material enthaltenden Formkörpers, das die folgenden Stufen umfaßt:
(I) Versetzen eines Gemischs enthaltend ein poröses oxidisches Material oder ein Gemisch aus zwei oder mehr davon mit einer Mischung enthaltend mindestens einen Alkohol und Wasser, und
(II) Kneten, Verformen, Trocknen und Calcinieren des gemäß Stufe (I) versetzten Gemischs.

Eines der Hauptthemen bzgl. des Eigenschaftsprofils der hier in Rede stehenden Formkörper sind zum einen eine für die Durchführung katalytischer Umsetzungen ausreichenden mechanischen Stabilität, sowie eine möglichst gute Aktivität und Selektivität im Hinblick auf das gewüschte Zielprodukt.

Insbesondere im Hinblick auf die Selektivität spielt die Auswahl des richtigen Bindemittels im Verformungsprozess eine grosse Rolle. Dies fällt insbesondere dann ins Gewicht, wenn Nebenreaktionen bestimmter Bindemittel unerwünscht sind und aus diesem Grund ganze Klassen von Bindemitteln, die einem derartigen Formkörper eine ausreichende Festigkeit verleihen könnten, z.B. aufgrund anderer negativer Eigenschaften nicht verwendet werden können. So wurde beispielsweise bislang angenommen, daß bei der Herstellung von Titansilicalit, der als Katalysator für die Epoxidation von z.B. Propylen mit Wasserstoffperoxid verwendet wird, aluminiumhaltige Bindemittel nicht verwendet werden, da es aufgrund der durch den aluminiumhaltigen Bindemittel induzierten Acidität zu vermehrter Ringöffnung und Nebenproduktbildung kommt (WO 98/55229).

EP 1 138 387 A1 offenbart ein Verfahren zur Herstellung eines Titansilicalitformkörpers durch
a) Ausbilden einer verformbaren Masse enthaltend Titansilicalit, ein Bindemittel und ein Anteigungsmittel, so dass die Curdkurve der verformbaren Masse einen Plateauwert im Bereich von 20 bis 90 mm aufweist,
b) Verformen der Masse aus Schritt a) zu einem Grünkörper,
c) eventuell Trocknen und
d) Calcinieren des Grünkörpers,
einen nach diesem Verfahren erhältlichen Titansilicalitformkörper und die Verwendung dieser Titansilicalitformkörper zur Epoxidierung von Olefinen oder Ammoximierungen von Ketonen.

Somit liegt der vorliegenden Erfindung die Aufgabe zugrunde, einen mindestens einen Zeolithen enthaltenden Formkörper bereitzustellen, der eine ausreichende mechanische Stabilität aufweist, um als Katalysator in einem Festbett verwendet zu werden, und darüberhinaus eine weiter verbesserte Aktivität und/oder Selektivität, z.B. bei der Epoxidation von organischen Verbindungen, insbesondere bei der Umsetzung von Propylen zu Propylenoxid, aufweisen sollte.

Dabei wurde nunmehr gefunden, daß gerade die Verwendung von aluminiumhaltigen Bindemitteln zu einem Formkörper führt, der die oben diskutierten Anforderungen erfüllt.

Demgemäß betrifft die vorliegende Erfindung ein Verfahren zur Herstellung eines mindestens einen Zeolithen enthaltenden und Meso- und Makroporen aufweisenden Formkörpers mit einem Gesamtporenvolumen der Meso- und Makroporen von 0,01 bis 1,5 ml/g, das die folgenden Stufen (I) bis (III) umfasst:
(I) Herstellen eines Gemischs, enthaltend einen Zeolithen oder ein Gemisch aus zwei oder mehr davon,
(II) Vermischen des in Stufe (I) erhaltenen Gemischs mit einem aluminiumhaltigen Bindemittel und mindestens einem Polymer zur Einstellung einer bestimmten Porengröße, Porengrößenverteilung und Porenvolumina, wobei der Gehalt an diesem Polymer 15 bis 75 Gew.-%, bezogen auf die Menge Zeolith im Gemisch, beträgt,
(III) Kneten, Verformen, Trocknen und Calcinieren des in Stufe (II) erhaltenen Gemischs,
dadurch gekennzeichnet, dass das mindestens eine Polymer zur Einstellung einer bestimmten Porengröße, Porengrößenverteilung und Porenvolumina eine in wässrigen Lösungsmitteln dispergier-, suspendier- oder emulgierbare polymere Vinylverbindung ist, sowie einen
Formkörper, enthaltend mindestens einen Zeolithen und Meso- und Makroporen mit einem Gesamtporenvolumen der Meso- und Makroporen von 0,01 bis 1,5 ml/g aufweisend, erhältlich durch ein Verfahren, das die folgenden Stufen (I) bis (III) umfasst:
(I) Herstellen eines Gemischs, enthaltend einen Zeolithen oder ein Gemisch aus zwei oder mehr davon,
(II) Vermischen des in Stufe (I) erhaltenen Gemischs mit einem aluminiumhaltigen Bindemittel und mindestens einem Polymer zur Einstellung einer bestimmten Porengröße, Porengrößenverteilung und Porenvolumina, wobei der Gehalt an diesem Polymer 15 bis 75 Gew.-%, bezogen auf die Menge Zeolith im Gemisch, beträgt,
(III) Kneten, Verformen, Trocknen und Calcinieren des in Stufe (II) erhaltenen Gemischs,
dadurch gekennzeichnet, dass das mindestens eine Polymer zur Einstellung einer bestimmten Porengröße, Porengrößenverteilung und Porenvolumina eine in wässrigen Lösungsmitteln dispergier-, suspendier- oder emulgierbare polymere Vinylverbindung ist.

Die erfindungsgemäße Herstellung der oben beschriebenen Formkörper ausgehend von einem Zeolithen in Pulverform beinhaltet die Bildung einer plastischen Masse, die mindestens einen Zeolithen, ein aluminiumhaltiges Bindemittel, einen Porenbildner auf der Basis von in wässrigen Lösungsmitteln dispergier-, suspendier- oder emulgierbaren Polymeren, gegebenenfalls eine Mischung enthaltend mindestens einen Alkohol und Wasser, gegebenenfalls eine oder mehrere organische viskositätssteigernde Substanzen und weitere aus dem Stand der Technik bekannte Zusatzstoffe enthält.

Die durch inniges Vermischen, insbesondere Kneten, der obigen Komponenten erhaltene plastische Masse wird vorzugsweise durch Strangpressen oder Extrudieren verformt und der erhaltene Formkörper wird nachfolgend getrocknet und abschließend calciniert.

Bezüglich der zur Herstellung des erfindungsgemäßen Formkörpers verwendbaren Zeolithe existieren keinerlei Beschränkungen. Vorzugsweise wird ein Titan-, Zirkonium-, Chrom-, Niob-, Eisen-, Bor- oder Vanadium-haltiger Zeolith und insbesondere ein Titansilicalit eingesetzt.

Zeolithe sind bekanntermaßen kristalline Allumosilicate mit geordneten Kanal- und Käfigstrukturen, die Mikroporen aufweisen. Die Begriffe "Mikroporen", wie er im Rahmen der vorliegenden Erfindung verwendet wird, entspricht der Definition in "Pure Appl. Chem." 45, S. 71 ff., insbesondere S. 79 (1976), und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W.M. Meier und D.H. Olson in "Atlas of Zeolithe Structure Types", Elsevier, 4. Auflage, London 1996.

Es sind nun auch Zeolithe bekannt, die kein Aluminiuin enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beschrieben, beispielsweise in der EP-A 0 311 983 oder EP-A 405 978. Außer Silicium und Titan können solche Materialien auch zusätzliche Elemente wie z. B. Aluminium, Zirkonium, Zinn, Eisen, Kobalt, Nickel, Gallium, Bor oder geringe Menge an Fluor enthalten. In den mit dem erfindungsgemäßen Verfahren vorzugsweise regenerierten Zeolith-Katalysatoren kann das Titan des Zeoliths teilweise oder vollständig durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe, insbesondere solche mit einer Kristallstruktur vom MFI-Typ, sowie Möglichkeiten zu ihrer Herstellung sind beispielsweise in der WO 98/55228, WO 98/03394, WO 98/03395, EP-A 0 311 983 oder der EP-A 0 405 978 beschrieben.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂₋Phasen unterscheiden.

Üblicherweise stellt man die genannten Titan-, Zirkonium-, Chrom-, Niob-, Eisen- und Vanadiumzeolithe dadurch her, daß man eine wässrige Mischung aus einer SiO₂-Quelle, einer Titan-, Zirkonium-, Chrom-, Niob-, Eisen bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen von basischen Verbindungen, in einem Druckbehälter unter erhöhter Temperatur im Zeitraum mehrerer Stunden oder einiger Tage umsetzt, wobei ein kristallines Produkt entsteht.

Das durch die hydrothermale Umsetzung erhaltene kristalline Produkt wird anschließend nach bekannten Methoden abfiltriert, zentrifugiert, sprühgetrocknet oder sprühgranuliert, vorzugsweise sprühgetrocknet oder sprühgranuliert, insbesondere sprühgranuliert unter Verwendung einer Wirbelschicht-Sprühgranulationstrocknung.

In einer weiteren Ausführungsform der Herstellung des Zeolithen kann der Feststoffgehalt des in Stufe (I) nach der hydrothermalen Kristallisation erhaltenen Produkts in Form einer Suspension erhöht werden. Dies kann beispielsweise durch Auftrennung der dort erhaltenen Suspension in mehrere Teile und anschließende Abtrennung des in diesem Teil enthaltenen Zeolithen durch kuchenbildende Filtration, Zentrifugation und andere geeignete Verfahren erreichen. Den so erhaltenen Filterkuchen oder das Sediment kann man anschließend, gegebenenfalls nach einem Waschschritt in dem verbliebenen Teil der Zeolith-Suspension suspendieren.

Der Einsatz der Sprühtrocknung oder Sprühgranulation führt insgesamt zu Granulaten des Zeolithen, die dann, wie weiter unten beschrieben, zu dem erfindungsgemäßen Formkörper umgesetzt werden können. Weitere Details bezüglich der hier in Rede stehenden Sprühtrocknung oder Sprühgranulation sind der DE- 197 31 627 bzw. der US 6 106 803 zu entnehmen.

In dem so erhaltenen Pulver liegt das Titan, bzw. das Zirkonium, Chrom, Niob, Eisen und/oder Vanadium zumindest teilweise innerhalb des Zeolithgerüsts in wechselndem Anteil mit 4-, 5- oder 6-facher Koordination vor. Zur Verbesserung der katalytischen Verhaltens kann sich noch eine mehrmalige Waschbehandlung mit schwefelsaurer Wasserstoffperoxidlösung anschließen, worauf das Titan- bzw. Zirkonium-, Chrom-, Niob-, Eisen-, Vanadiumzeolith-Pulver erneut getrocknet und gebrannt werden muß; daran kann sich eine Behandlung mit Alkalimetallverbindungen anschließen, um den Zeolith von der H-Form in die Kation-Form zu überführen. Das so hergestellte Titan- bzw. Zirkonium-, Chrom-, Niob-, Eisen-, Vanadiumzeolith-Pulver wird dann, wie nachstehend beschrieben, zu einem Formkörper verarbeitet.

Dabei sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Cerüststruktur zu nennen.

Als Bindemittel zur Herstellung des erfindungsgemäßen Formkörpers werden aluminiumhaltige Bindemittel eingesetzt. Zu nennen sind hierbei insbesondere Aluminiumoxide, wie z. B. alpha-, beta-, gamma-, delta-, eta-, kappa-, chi- und theta-Aluminiumoxid und deren anorganische oder metallorganische Vorläuferverbindungen, wie z.B. Gibbsit, Bayerit, Boehmit, Pseudoboehmit, Trialkoxyaluminate, bevorzugt Aluminiumtriisopropylat.

Diese aluminiumhaltigen Bindemittel können entweder alleine, als Gemisch aus zwei oder mehr davon, oder zusammen mit bislang für zeolithische Materialien eingesetzen Bindemitteln, wie z.B. Oxiden des Siliziums, Bors, Phosphors, Zirkoniums und/oder Titans verwendet werden. Im einzelnen sind diesbezüglich insbesondere Siliziumdioxid, wobei das SiO₂ als Kieselsol oder in Form von Tetraalkoxysilanen in den Formgebungsschritt eingebracht werden kann sowie Tone, z.B. Montmorillonite, Kaoline, Bentonite, Halloysite, Dickite, Nacrite und Ananxite zu nennen.

Vorzugsweise wird als Bindemittel jedoch ein Metallsäureester des Aluminiums oder ein Gemisch aus zwei oder mehr davon als Bindemittel in Stufe (II) des erfindungsgemäßen Verfahrens zugesetzt.

Zusätzlich zu den oben genannten Bindemittelkornponenten sind ferner Orthokieselsäureester, Tetraalkoxysilane, Tetraalkoxytitanate, Tetraalkoxyzirkonate oder ein Gemisch aus zwei oder mehr davon, vorzugsweise Tetramethoxysilan, Tetraethoxysilan, Tetrapropoxysilan und Tetrabutoxysilan, die analogen Tetraalkoxytitan- und -zirkonium-Verbindungen sowie Trimethoxy-, Triethoxy, Tripropoxy, wobei Tetramethoxysilan und Tetraethoxysilan besonders bevorzugt sind, zu nennen.

Der erfindungsgemäße Formkörper enthält vorzugsweise bis zu ungefähr 80 Gew.-%, weiter bevorzugt ungefähr 10 bis ungefähr 75 Gew.-% und insbesondere ungefähr 25 bis ungefähr 45 Gew.-% Bindemittel, jeweils bezogen auf die Gesamtmasse des Formkörpers, wobei die Menge an Bindemittel sich aus dem entstehenden Aluminiumoxid ergibt.

Wie sich aus obigem bereits ergibt, können selbstverständlich auch Gemische aus zwei oder mehr der obengenannten Bindemittel eingesetzt werden.

Der erfindungsgemäße Formkörper kann Mikro-, Meso- und Makroporen aufweisen, wobei die Definition der Begriffe "Mesoporen" und "Makroporen" ebenfalls derjenigen in oben erwähnter Literatur gemäß Pure Appl. Chem. entspricht und Poren mit einem Durchmesser von > 2 nm bis ca. 50 nm (Mesoporen) bzw. > ungefähr 50 nm (Makroporen) bezeichnet.

Der erfindungsgemäße Formkörper weist Meso- und Makroporen auf. Vorzugsweise handelt es sich dabei um Formkörper, deren Gesamtporenvolumen der Meso- und Makroporen im Bereich von 0,01 bis 1,5 ml/g, vorzugsweise von 0,3 bis 1,1 ml/g und insbesondere bevorzugt von 0,6 bis 0,95 ml/g beträgt.

Im Rahmen des erfindungsgemäßen Verfahrens werden demgemäß zur Einstellung einer bestimmten Porengröße, Porengrößenverteilung und Porenvolumina noch Polymere zugegeben wobei erfindungsgemäß in wässrigen Lösungsmitteln dispergier-, suspendier- oder emulgierbare Polymere für diesen Zweck eingesetzt werden.

Dabei wird das Polymer vorzugsweise ausgewählt unter polymeren Vinylverbindungen, wie z.B. Polystyrol, Polyacrylaten, Polymethacrylaten, Polyolefinen, Polyamiden und Polyestern. Diese Polymere werden beim Calcinieren wieder weitgehend aus dem Formkörper entfernt und hinterlassen Poren der obengenannten Art.

Der Gehalt an Polymer während der Herstellung des Formkörpers beträgt ungefähr 15 bis ungefähr 75 Gew.-% und insbesondere ungefähr 25 bis 55 Gew.-%, jeweils bezogen auf die Menge an Zeolith im Gemisch.

Ferner wird bei der Herstellung des erfindungsgemäßen Formkörpers ein Anteigungsmittel eingesetzt.

Als Anteigungsmittel können alle dafür geeigneten, aus dem Stand der Technik bekannten Substanzen verwendet werden. Vorzugsweise sind dies organische, insbesondere hydrophile Polymere, wie z.B. Cellulose, Stärke, Polyacrylate, Polymethacrylate, Polyvinylalkohol, Polyvinylpyrrolidon, Polyisobuten, Polytetrahydrofuran. Diese Substanzen fördern in erster Linie die Bildung einer plastischen Masse während des Knet-, Verformungs- und Trocknungsschritts durch Verbrücken der Primärpartikel und gewährleisten darüber hinaus die mechanische Stabilität des Formkörpers beim Verformen und Trocknen. Diese Substanzen werden beim Calcinieren wieder aus dem Formkörper entfernt.

Als weitere Zusatzstoffe können Amine oder aminartige Verbindungen, wie z.B. Tetraalkylammoniumverbindungen oder Aminoalkohole, sowie carbonathaltige Substanzen, wie z.B. Calciumcarbonat, zugesetzt werden. Derartige weitere Zusatzstoffe sind in EP-A 0 389 041, EP-A 0 200 260 und in WO 95/19222 beschrieben.

Statt basischer Zusatzstoffe ist es auch möglich saure Zusatzstoffe zu verwenden. Diese können unter anderem eine schnellere Reaktion des Metallsäureesters mit dem porösen oxidischen Material bewirken. Bevorzugt sind organische saure Verbindungen, die sich nach dem Verformungsschritt durch Calcinieren herausbrennen lassen. Besonders bevorzugt sind Carbonsäuren, wie z. B. Ameisensäure. Derartige Säuren modifizieren ebenfalls die Oberflächen der hier in Rede stehenden Formkörper.

Es können weitere Zuschlagstoffe und Lösungsmittel eingesetzt werden, die zur Plastifizierung der zu formenden Masse beitragen. Derartige Lösungsmittel und Zuschlagstoffe sind dem Fachmann bekannt.

Selbstverständlich können auch Gemische aus zwei oder mehr der oben genannten Zusatzstoffe eingesetzt werden.

Die Zugabereihenfolge der Bestandteile der den Zeolithen enthaltenden Masse (Gemisch) ist nicht kritisch. Es ist sowohl möglich, zuerst das Bindemittel zuzugeben, anschließend das in Wasser dispergierbare, emulgierbare oder suspendierbare Polymer, die organische viskositätssteigernde Substanz, ggf. den Zusatzstoff und zum Schluß das Anteigungsmittel, als auch die Reihenfolge bezüglich des Bindemittels, Polymers, der organischen viskositätssteigernden Substanz und der Zusatzstoffe zu vertauschen.

Nach der Zugabe des Bindemittels zum pulverförmigen porösen Oxid, dem gegebenenfalls die organische viskositätssteigernde Substanz bereits zugegeben worden ist, wird die in der Regel noch pulverförmige Masse 10 bis 180 Minuten im Kneter oder Extruder homogenisiert. Dabei wird in der Regel bei Temperaturen im Bereich von ungefähr 10°C bis zum Siedepunkt des Anteigungsmittel und Normaldruck oder leichtem überatmosphärischem Druck gearbeitet. Danach erfolgt die Zugabe der restlichen Bestandteile, und das so erhaltene Gemisch wird solange geknetet, bis eine verstrangbare oder extrudierfähige, plastische Masse entstanden ist.

Prinzipiell können für die Knetung und die Verformung alle herkömmlichen Knet- und Verformungsvorrichtungen bzw. Verfahren, wie sie zahlreich aus dem Stand der Technik für die Herstellung von z.B. Katalysator-Formkörpern bekannt sind, eingesetzt werden.

Wie bereits angedeutet, sind jedoch Verfahren bevorzugt, bei denen die Verformung durch Extrusion in üblichen Extrudern, beispielsweise zu Strängen mit einem Durchmesser von üblicherweise ungefähr 1 bis ungefähr 10 mm, insbesondere ungefähr 2 bis ungefähr 5 mm, erfolgt. Derartige Extrusionsvorrichtungen werden beispielsweise in Ullmann's Enzyklopädie der Technischen Chemie, 4. Auflage, Bd. 2, S. 295 ff., 1972 beschrieben. Neben der Verwendung eines Extruders wird ebenfalls vorzugsweise eine Strangpresse zur Verformung verwendet.

Nach Beendigung des Strangpressens oder Extrudierens werden die erhaltenen Formkörper bei im allgemeinen ungefähr 30 °C bis 140 C (1 bis 20 h, Normaldruck) getrocknet und bei ungefähr 400 °C bis ungefähr 800 °C (3 bis 10 h, Normaldruck) calciniert.

Selbstverständlich können die erhaltenen Stränge bzw. Extrudate zerkleinert werden. Sie werden dabei vorzugsweise zu einem Granulat oder Splitt mit einem Partikeldurchmesser von 0,1 bis 5 mm, insbesondere 0,5 bis 2 mm zerkleinert.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Die erfindungsgemäßen bzw. nach dem erfindungsgemäßen Verfahren hergestellten Formkörper besitzen - verglichen mit entsprechenden Formkörpern des Standes der Technik - eine gute mechanische Stabilität bei gleichzeitiger verbesserter Aktivität und/oder Selektivität.

Die erfindungsgemäßen bzw. erfindungsgemäß hergestellten Formkörper können zur katalytischen Umwandlung organischer Moleküle eingesetzt werden. Umsetzungen dieser Art sind beispielsweise Oxidationen, die Epoxidation von Olefinen wie z.B. die Herstellung von Propylenoxid aus Propylen und H₂O₂, die Hydroxylierung von Aromaten, wie z.B. Hydrochinon aus Phenol und H₂O₂ oder Toluol zu Kresol, die Umwandlung von Alkanen zu Alkoholen, Aldehyden und Säuren, Isomerisierungsreaktionen, wie z.B. die Umwandlung von Epoxiden zu Aldehyden, sowie weitere in der Literatur mit derartigen Formkörpern, insbesondere Zeolith-Katalysatoren beschriebenen Umsetzungen, wie sie beispielsweise in W. Hölderich, "Zeolites: Catalysts for the Synthesis of Organic Compounds", Elsevier, Stud. Surf. Sci. Catal., 49, Amsterdam (1989), S. 69 bis 93, und insbesondere für mögliche Oxidationsreaktionen von B. Notari in Stud. Surf. Sci. Catal., 37 (1987), S. 413 bis 425, beschrieben sind.

Dabei eignen sich die vorstehend ausführlich diskutierten Zeolithe insbesondere für die Epoxidation von Olefinen, vorzugsweise solchen mit 2 bis 8 C-Atomen, weiter bevorzugt Ethylen, Propylen oder Buten, und insbesondere Propen zu den entsprechenden Olefinoxiden. Demgemäß betrifft die vorliegende Erfindung insbesondere die Verwendung des hierin beschriebenen Formkörpers zur Herstellung von Propylenoxid ausgehend von Propylen und Wasserstoffperoxid.

Demgemäß betrifft die vorliegende Erfindung die Verwendung des erfindungsgemäßen bzw. erfindungsgemäß hergestellten Formkörpers oder eines Gemischs aus zwei oder mehr davon zur Epoxidation von organischen Verbindungen mit mindestens einer C-C-Doppelbindung, zur Hydroxylierung von aromatischen organischen Verbindungen, zur Umwandlung von Alkanen zu Alkoholen, Ketonen, Aldehyden und Säuren, zur Ammonoximierung von Ketonen und zur Herstellung von Amin-N-Oxiden, insbesondere die Verwendung des Formkörpers oder eines Gemischs aus zwei oder mehr davon zur Epoxidation eines Olefins, weiter bevorzugt zur Herstellung von Propylenoxid ausgehend von Propylen und Wasserstoffperoxid.

Bedingt durch die erfindungsgemäßen Maßnahmen bei der Herstellung der Formkörper, nämlich die Verwendung eines aluminiumhaltigen Bindemittels in Kombination mit einem in wässrigen Lösungsmitteln dispergier-, suspendier- oder emulgierbaren Polymer, bewirkt eine Erhöhung des Gesamtporenvolumens des erhaltenen Formkörpers, wodurch sich u. a. eine Verbesserung der Katalysatoreigenschaften ergibt.

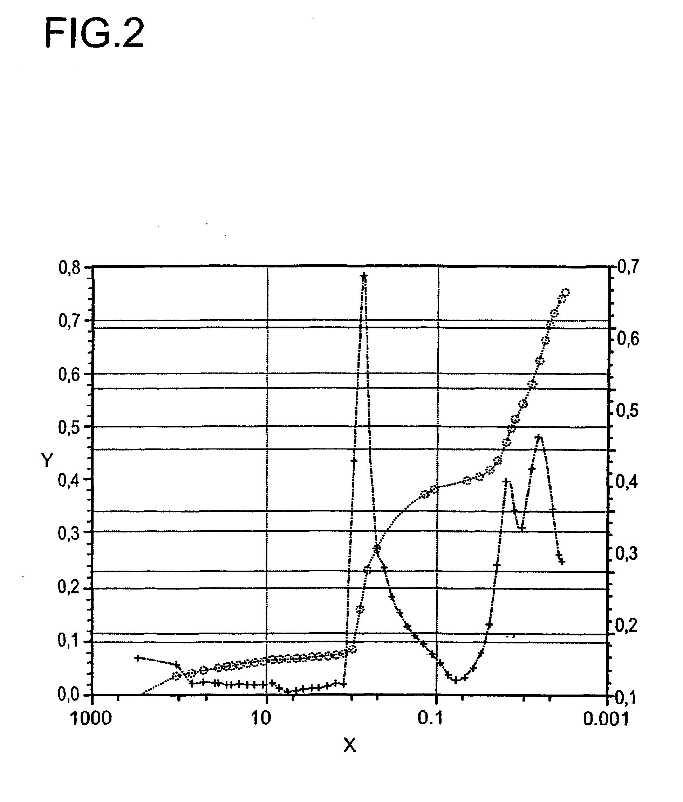

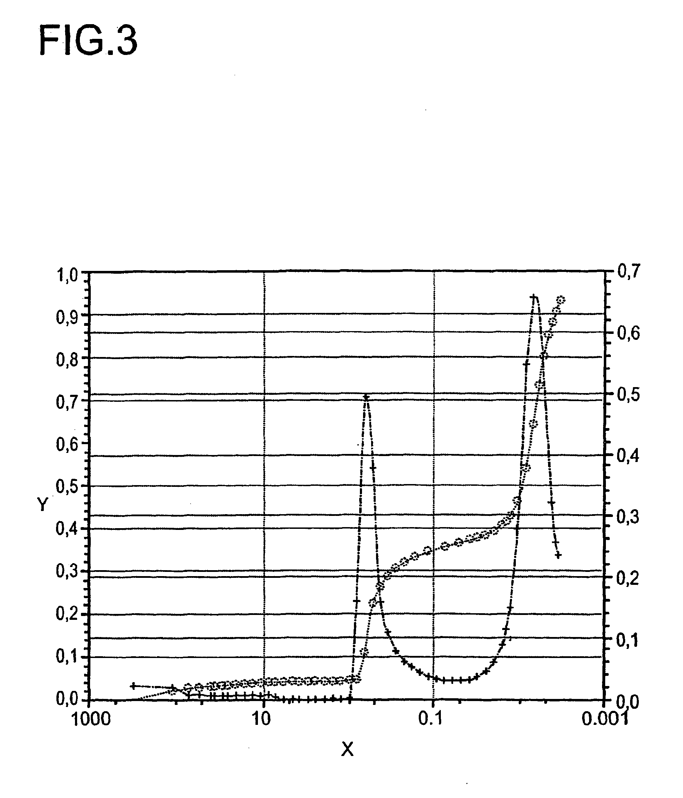

Die Porenverteilung der erhaltenen Formkörper ist aus den der Anmeldung beiliegenden Figuren 1 bis 3 ersichtlich.

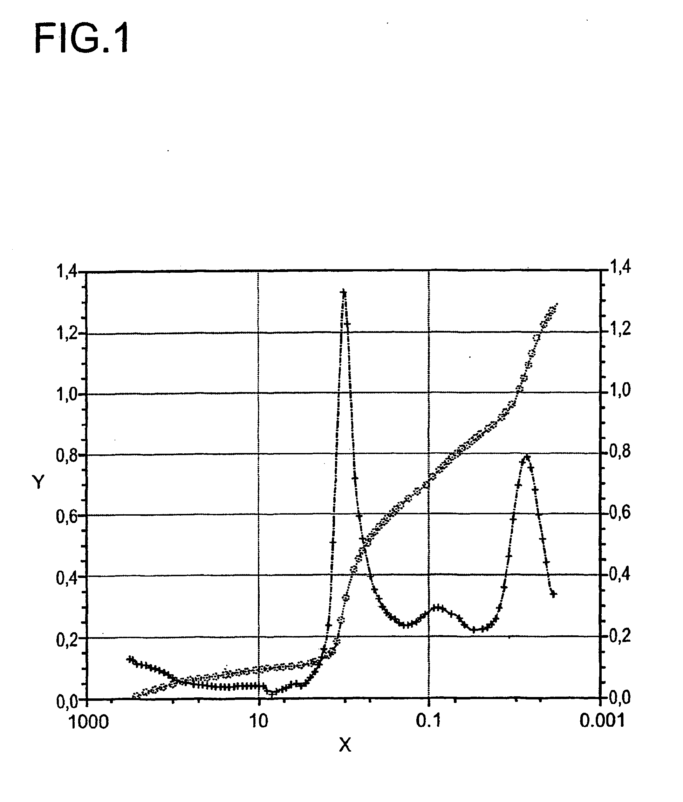

Dabei gibt Fig. 1 die Ergebnisse der Quecksilber-Porosimetrie-Analyse des Formkörpers gemäß Beispiel 2 wieder.

Fig. 2 gibt das Ergebnis der Quecksilber-Porosimetrie-Analyse des Formkörpers gemäß Beispiel 3 wieder.

Fig. 3 gibt das Ergebnis der Quecksilber-Porosimetrie-Analyse des Formkörpers gemäß Beispiel 4 wieder, und betrifft somit ebenfalls den technischen Hintergrund der vorliegenden Erfindung.

Dabei ist auf der x-Achse der Porendurchmesser in Mikrometer, und auf der y-Achse der Logarithmus des Porenvolumens in ml/g aufgetragen. Die mit "x" gezeichnete Kurve entspricht dem differenziellen Porenvolumen. Die mit "o" gezeichnete Kurve entspricht dem kumulativen Porenvolumen.

### BEISPIELE

### Beispiel 1

In einem Vierhalskolben (41 Inhalt) wurden 910 g Tetraethylorthosilicat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Anschließend versetzte man mit 1600 g einer 20 gew.-%igen Tetrapropylammoniumhydroxid-Lösung (Alkaligehalt < 10 ppm) und rührte noch eine Stunde nach. Bei 90 °C bis 100 °C wurde das aus der Hydrolyse gebildete Alkoholgemisch (ca. 900 g) abdestilliert. Man füllte mit 31 Wasser auf und gab das mittlerweile leicht opaque Sol in einen 51 fassenden Rührautoklaven aus Edelstahl.

Mit einer Heizrate von 3 °C/min wurde der verschlossene Autoklav (Ankerrührer, 200 U/min) auf eine Reaktionstemperatur von 175 °C gebracht. Nach 92 h war die Reaktion beendet. Das erkaltete Reaktionsgemisch (weiße Suspension) wurde abzentrifugiert und mehrfach mit Wasser neutral gewaschen. Der erhaltene Feststoff wurde bei 110 °C innerhalb von 24 h getrocknet (Auswaage: 298 g).

Anschließend wurde unter Luft bei 550 °C in 5 h das im Zeolithen verbliebene Templat abgebrannt. (Calcinierungsverlust: 14 Gew.-%).

Das reinweiße Produkt hatte nach naßchemischer Analyse einen Ti-Gehalt von 1,5 Gew.-% und einen Gehalt an Restalkali unterhalb 100 ppm. Die Ausbeute auf eingesetztes SiO₂ betrug 97 %. Die Kristallite hatten eine Größe von 0,05 bis 0,25 µm und das Produkt zeigte im IR eine typische Bande bei ca. 960 cm⁻¹.

### Beispiel 2

Das getrocknete und bei 500 °C für 5 h an Luft calcinierte Titansilikat-Pulver aus Beispiel 1 (60 g) wurde mit 40 g einer Aluminiumoxid-Quelle (Versal 250), 2 g Ameisensäure und insgesamt 110 ml einer wäßrigen Polymerdispersion (32 Gew.-% Polystyrol emulgiert in Wasser) in einem Kneter für eine Dauer von 65 min gemischt und die plastifizierte Masse bei einem Druck von 60 bar über eine Matrize zu Strängen mit einem Durchmesser von 1,5 mm ausgeformt. Die Ausbeute betrug 74 g. Abschließend wurde das ausgeformte Material bei 120 °C für 4 h getrocknet und bei 470 °C für 5 h unter Luft calciniert, wobei das Polymer entfernt wurde und Poren hinterließ.

Mittels Quecksilberporosimetrie (DIN 66133) wurde ein Gesamtporenvolumen von 1,29 ml/g gemessen. Der häufigste Durchmesser der Makroporen lag bei 1,1 µm, der der Mesoporen bei 79 nm und 7,5 nm.

### Beispiel 3

Das getrocknete und bei 500 °C für 5 h an Luft calcinierte Titansilikat-Pulver aus Beispiel 1 (60 g) wurde mit 40 g einer Aluminiumoxid-Quelle (Versal 250), 2 g Ameisensäure und insgesamt 60 ml einer wäßrigen Polymerdispersion (32 Gew.-% Polystyrol emulgiert in Wasser) in einem Kneter für eine Dauer von 45 min gemischt und bei einem Druck von 65 bar über eine Matrize zu Strängen mit einem Durchmesser von 1,5 mm ausgeformt. Die Ausbeute betrug 73 g. Abschließend wurde das ausgeformte Material bei 120 °C für 4 h getrocknet und bei 470 °C für 5 h unter Luft calciniert, wobei das Polymer entfernt wurde und Poren hinterließ.

Mittels Quecksilberporosimetrie wurde ein Gesamtporenvolumen von 0,67 ml/g gemessen. Der häufigste Durchmesser der Makroporen lag bei 0,78 µm, der der Mesoporen bei 16 bzw. 7 nm.

### Vergleichsbeispiel

Das Vergleichsbeispiel betriftt den allgemeinen technischen Hintergrund des erfindung.

Das getrocknete und bei 500 °C für 5 h an Luft calcinierte Titansilikat-Pulver aus Beispiel 1 (60 g) wurde mit 40 g einer Aluminiumoxid-Quelle (Versal 250), 2 g Ameisensäure und insgesamt 64 ml Wasser in einem Kneter für eine Dauer von 42 min gemischt und bei einem Druck von 55 bar über eine Matrize zu Strängen mit einem Durchmesser von 1,5 mm ausgeformt. Die Ausbeute betrug 68 g. Abschließend wurde das ausgeformte Material bei 120 °C für 4 h getrocknet und bei 470 °C für 5 h unter Luft calciniert.

Mittels Quecksilberporosimetrie wurde ein Gesamtporenvolumen von 0,62 ml/g gemessen. Der häufigste Durchmesser der Makroporen lag bei 0,64 µm, der der Mesoporen bei 7 nm.

### Beispiel 5

Der Katalysator aus Beispiel 2 wurde zur Herstellung von Propylenoxid ausgehend von Propen in Gegenwart von H₂O₂ eingesetzt. Die verwendeten Reaktionsbedingungen entsprechen denen der Beispiele 4 bis 8 der WO 98/55229.

Dabei liefert der Katalysator gemäß Beispiel 2 eine Ausbeute an Propylenoxid von 4,5 Gew.-%, Katalysator gemäß Beispiel 3 liefert eine Propylenoxid-Ausbeute von 4,1 Gew.-%, und der Katalysator gemäß dem Vergleichsbeispeil liefert eine Propylenoxid-Ausbeute von 3,9 Gew.-%.

## Patentansprüche

1. Verfahren zur Herstellung eines mindestens einen Zeolithen enthaltenden und Meso- und Makroporen aufweisenden Formkörpers mit einem Gesamtporenvolumen der Meso- und Makroporen von 0,01 bis 1,5 ml/g, das die folgenden Stufen (I) bis (III) umfasst:
(I) Herstellen eines Gemischs, enthaltend einen Zeolithen oder ein Gemisch aus zwei oder mehr davon,
(II) Vermischen des in Stufe (I) erhaltenen Gemischs mit einem aluminiumhaltigen Bindemittel und mindestens einem Polymer zur Einstellung einer bestimmten Porengröße, Porengrößenverteilung und Porenvolumina, wobei der Gehalt an diesem Polymer 15 bis 75 Gew.-%, bezogen auf die Menge Zeolith im Gemisch, beträgt,
(III) Kneten, Verformen, Trocknen und Calcinieren des in Stufe (II) erhaltenen Gemischs,
**dadurch gekennzeichnet, dass** das mindestens eine Polymer zur Einstellung einer bestimmten Porengröße, Porengrößenverteilung und Porenvolumina eine in wässrigen Lösungsmitteln dispergier-, suspendier- oder emulgierbare polymere Vinylverbindung ist.

2. Verfahren nach Anspruch 1, **dadurch gekennzeichnet, dass** der mindestens eine Porenbildner ausgewählt wird aus der Gruppe bestehend aus Polystyrol, Polyacrylaten, Polymethacrylaten, Polyolefinen, Polyamiden, Polyestern und einem Gemisch aus zwei oder mehr davon.

3. Verfahren nach Anspruch 1 oder 2, **dadurch gekennzeichnet, dass** das aluminiumhaltige Bindemittel ausgewählt wird aus der Gruppe bestehend aus Aluminiumoxid, einer organischen Aluminiumoxid-Vorläuferverbindung, einer metallorganischen Aluminiumoxid-Vorläuferverbindung und einem Gemisch aus zwei oder mehr davon.

4. Verfahren nach Anspruch 3, **dadurch gekennzeichnet, dass** das aluminiumhaltige Bindemittel ausgewählt wird aus der Gruppe bestehend aus Trialkoxyaluminaten, Boehmit, Pseudoboehmit und einem Gemisch aus zwei oder mehr davon.

5. Verfahren nach einem der Ansprüche 1 bis 4, **dadurch gekennzeichnet, dass** das in der Stufe (II) erhaltene Gemisch in Stufe (III) durch Agglomerieren, Tablettieren, Strangpressen oder Extrudieren verformt wird.

6. Verfahren nach einem der Ansprüche 1 bis 5, **dadurch gekennzeichnet, dass** der mindestens eine Zeolith ausgewählt wird aus der Gruppe bestehend aus Titan-, Zirkonium-, Chrom-, Niob-, Eisen-, Bor-, Vanadium-haltigem Zeolith und einem Gemisch aus zwei oder mehr davon.

7. Formkörper, enthaltend mindestens einen Zeolithen und Meso- und Makroporen mit einem Gesamtporenvolumen der Meso- und Makroporen von 0,01 bis 1,5 ml/g aufweisend, erhältlich durch ein Verfahren, das die folgenden Stufen (I) bis (III) umfasst:
(I) Herstellen eines Gemischs, enthaltend einen Zeolithen oder ein Gemisch aus zwei oder mehr davon,
(II) Vermischen des in Stufe (I) erhaltenen Gemischs mit einem aluminiumhaltigen Bindemittel und mindestens einem Polymer zur Einstellung einer bestimmten Porengröße, Porengrößenverteilung und Porenvolumina, wobei der Gehalt an diesem Polymer 15 bis 75 Gew.-%, bezogen auf die Menge Zeolith im Gemisch, beträgt,
(III) Kneten, Verformen, Trocknen und Calcinieren des in Stufe (II) erhaltenen Gemischs,
**dadurch gekennzeichnet, dass** das mindestens eine Polymer zur Einstellung einer bestimmten Porengröße, Porengrößenverteilung und Porenvolumina eine in wässrigen Lösungsmitteln dispergier-, suspendier- oder emulgierbare polymere Vinylverbindung ist.

8. Verwendung eines Formkörpers gemäß Anspruch 7 oder eines Formkörpers, erhältlich durch ein Verfahren gemäß einem der Ansprüche 1 bis 6, oder eines Gemischs aus zwei oder mehr davon zur Epoxidation von organischen Verbindungen mit mindestens einer C-C-Doppelbindung, zur Hydroxylierung von aromatischen organischen Verbindungen, zur Umwandlung von Alkanen in Alkohole, Ketone, Aldehyde und Säuren, zur Ammonoximierung von Ketonen und zur Herstellung von Amin-N-Oxiden.

9. Verwendung mindestens einer, in wässrigen Lösungsmitteln dispergier-, suspendier-oder emulgierbaren polymeren Vinylverbindung, ausgewählt aus der Gruppe bestehend aus Polystyrol, Polyacrylaten, Polymethacrylaten, Polyolefinen, Polyamiden, Polyestern und einem Gemisch aus zwei oder mehr davon zur Einstellung einer bestimmten Porengröße, Porengrößenverteilung und Porenvolumina, zur Herstellung eines Meso- und Makroporen aufweisenden Formkörpers gemäß Anspruch 7 mit einem Gesamtporenvolumen der Meso- und Makroporen von 0,01 bis 1,5 ml/g, enthaltend mindestens einen Zeolithen, wobei der Gehalt an eingesetzter polymerer Vinylverbindung 15 bis 75 Gew.-%, bezogen auf die Menge Zeolith, beträgt.

## Claims

1. A process for the production of a molding comprising at least one zeolite and mesopores and macropores, the molding having a total pore volume of the mesopores and macropores of from 0.01 to 1.5 ml/g, which process comprises the following stages (I) to (III):
(I) preparation of a mixture comprising a zeolite or a mixture of two or more thereof,
(II) mixing of the mixture obtained in stage (I) with an aluminum-containing binder, and at least one polymer for establishing a specific pore size, pore size distribution and pore volume, wherein the content in this polymer is from 15 to 75% by weight, based on the amount of zeolite in the mixture.
(III) kneading, molding, drying and calcination of the mixture obtained in stage (II),
wherein the at least one polymer for establishing a specific pore size, pore size distribution and pore volume is a polymeric vinyl compound dispersible, suspendable or emulsifiable in aqueous solvents.

2. The process according to claim 1, wherein the at least one pore former is selected from the group consisting of polystyrene, polyacrylates, polymethacrylates, polyolefins, polyamides, polyesters and a mixture of two or more thereof.

3. The process according to claim 1 or 2, wherein the aluminum-containing binder is selected from the group consisting of alumina, an organic alumina precursor compound, an organometallic alumina precursor compound and a mixture of two or more thereof.

4. The process according to claim 3, wherein the aluminum-containing binder is selected from the group consisting of trialkoxyaluminates, boehmite, pseudoboehmite and a mixture of two or more thereof.

5. The process according to any of claims 1 to 4, wherein the mixture obtained in stage (II) is molded by agglomeration, tableting, processing in an extrusion press or extrusion in stage (III).

6. The process according to any of claims 1 to 5, wherein the at least one zeolite is selected from the group consisting of titanium-, zirconium-, chromium-, niobium-, iron-, boron- or vanadium-containing zeolite, and a mixture of two or more thereof.

7. A molding comprising at least one zeolite and mesopores and macropores, the molding having a total pore volume of the mesopores and macropores of from 0.01 to 1.5 ml/g and obtainable by a process which comprises the following stages (I) to (III):
(I) preparation of a mixture comprising a zeolite or a mixture of two or more thereof,
(II) mixing of the mixture obtained in stage (I) with an aluminum-containing binder and at least one polymer for establishing a specific pore size, pore size distribution and pore volume, wherein the content in this polymer is from 15 to 75% by weight, based on the amount of zeolite in the mixture.
(III) kneading, molding, drying and calcination of the mixture obtained in stage (II),
wherein the at least one polymer for establishing a specific pore size, pore size distribution and pore volume is a (polymeric) vinyl compound dispersible, suspendable or emulsifiable in aqueous solvents.

8. The use of a molding according to claim 7 or of a molding produced by a process according to any of claims 1 to 6 or of a mixture of two or more thereof for the epoxidation of organic compounds having at least one C-C double bond, for the hydroxylation of aromatic organic compounds, for the conversion of alkanes into alcohols, ketones, aldehydes and acids, for the ammoximation of ketones and for the preparation of amine N-oxides.

9. The use of at least one polymeric vinyl compound dispersible, suspendable or emulsifiable in aqueous solvents, selected from the group consisting of polystyrene, polyacrylates, polymethacrylates, polyolefins, polyamides, polyesters and a mixture of two or more thereof for establishing a specific pore size, pore size distribution and pore volume, for the production of a molding having mesopores and macropores according to claim 7, having a total pore volume of the mesopores and macropores of from 0.01 to 1.5 ml/g, comprising at least one zeolite, wherein the content of polymeric vinyl compound used is from 15 to 75 % by weight, based on the amount of zeolite.

## Revendications

1. Procédé de préparation d'un corps façonné contenant au moins une zéolite et présentant des mésopores et macropores avec un volume poreux global des mésopores et macropores de 0,01 à 1,5 ml/g, qui comprend les étapes (I) à (III) suivantes :
(I) une préparation d'un mélange contenant une zéolite ou un mélange de deux ou de plusieurs d'entre elles,
(II) un mélange du mélange obtenu dans l'étape (I) avec un liant contenant de l'aluminium et au moins un polymère prévu pour ajuster une taille de pore, une répartition des tailles de pore et un volume poreux déterminés, la teneur en ce polymère étant de 15 à 75 % en poids dans le mélange, par rapport à la quantité de zéolite,
(III) un pétrissage, une déformation, un séchage et une calcination du mélange obtenu dans l'étape (II),
**caractérisé en ce que** ledit au moins un polymère prévu pour ajuster une taille de pore, une répartition des tailles de pore et un volume poreux déterminés est un composé vinylique polymère que l'on peut disperser, mettre en suspension ou émulsionner dans des solvants aqueux.

2. Procédé suivant la revendication 1, **caractérisé en ce que** ledit au moins un agent porogène est choisi parmi le groupe constitué du polystyrène, des polyacrylates, des polyméthacrylates, des polyoléfines, des polyamides, des polyesters et d'un mélange de deux ou de plusieurs de ces substances.

3. Procédé suivant la revendication 1 ou 2, **caractérisé en ce que** le liant contenant de l'aluminium est choisi parmi le groupe constitué de l'oxyde d'aluminium, d'un composé précurseur organique d'oxyde d'aluminium, d'un composé précurseur organométallique d'oxyde d'aluminium et d'un mélange de deux ou de plusieurs de ces substances.

4. Procédé suivant la revendication 3, **caractérisé en ce que** le liant contenant de l'aluminium est choisi parmi le groupe constitué des trialcoxyaluminates, de la boehmite, de la pseudoboehmite et d'un mélange de deux ou de plusieurs de ces substances.

5. Procédé suivant l'une des revendications 1 à 4, **caractérisé en ce que** le mélange obtenu dans l'étape (II) est déformé dans l'étape (III) par agglomération, production de comprimés, boudinage ou extrusion.

6. Procédé suivant l'une des revendications 1 à 5, **caractérisé en ce que** ladite au moins une zéolite est choisie parmi le groupe constitué de zéolites contenant du titane, du zirconium, du chrome, du niobium, du fer, du bore, du vanadium et d'un mélange de deux ou de plusieurs de ces substances.

7. Corps façonnés contenant au moins une zéolite et présentant des mésopores et macropores avec un volume poreux global des mésopores et macropores de 0,01 à 1,5 ml/g, que l'on peut obtenir par un procédé qui comprend les étapes suivantes (I) à (III) :
(I) une préparation d'un mélange contenant une zéolite ou un mélange de deux ou de plusieurs d'entre elles,
(II) un mélange du mélange obtenu dans l'étape (I) avec un liant contenant de l'aluminium et au moins un polymère prévu pour ajuster une taille de pore, une répartition des tailles de pore et un volume poreux déterminés, la teneur en ce polymère étant de 15 à 75 % en poids dans le mélange, par rapport à la quantité de zéolite,
(III) un pétrissage, une déformation, un séchage et une calcination du mélange obtenu dans l'étape (II),
**caractérisé en ce que** ledit au moins un polymère prévu pour ajuster une taille de pore, une répartition des tailles de pore et un volume poreux déterminés est un composé vinylique polymère que l'on peut disperser, mettre en suspension ou émulsionner dans des solvants aqueux.

8. Utilisation d'un corps façonné suivant la revendication 7, ou d'un corps façonné que l'on peut obtenir par un procédé suivant l'une des revendications 1 à 6, ou d'un mélange de deux ou de plusieurs d'entre eux, pour l'époxydation de composés organiques ayant au moins une double liaison C-C, pour l'hydroxylation de composés organiques aromatiques, pour la transformation d'alcanes en alcools, cétones, aldéhydes et acides, pour l'ammonoximation de cétones et pour la préparation d'amine-N-oxydes.

9. Utilisation d'au moins un composé vinylique polymère que l'on peut disperser, mettre en suspension ou émulsionner dans des solvants aqueux et qui est choisi parmi le groupe constitué du polystyrène, des polyacrylates, des polyméthacrylates, des polyoléfines, des polyamides, des polyesters et d'un mélange de deux ou de plusieurs de ces substances pour l'ajustement d'une taille de pore, d'une répartition des tailles de pore et d'un volume poreux déterminés, pour la préparation d'un corps façonné présentant des mésopores et des macropores selon la revendication 7 avec un volume poreux global des mésopores et macropores de 0,01 à 1,5 ml/g et contenant au moins une zéolite, la teneur en composé vinylique polymère mis en oeuvre étant de 15 à 75 % en poids par rapport à la quantité de zéolite.
